# EUROPEAN PATENT APPLICATION

(11) **EP 3 150 254 A1**
(43) Date of publication of application: **05.04.2017**
(21) Application number: 16190604.5
(22) Date of filing: 26.09.2016
(51) Int. Cl.: A61N 5/06

(54) **DW PERSONAL LASER**

(30) Priority: 29.09.2015 US 201514868549
(71) Applicant: Wentz, Deborah Rae, Wallowa, Oregon 97885 (US)
(72) Inventor: Wentz, Deborah Rae, Wallowa, Oregon 97885 (US)
(74) Representative: Romano, Giuseppe

(57) **Abstract**

The present invention provides laser therapy for treatment of herpes simplex virus infection also known as cold sores. The invention uses laser, preferably diode lasers which are directed against the infected area including rashes. The laser could provide immediate relief in pain and promote healing process. The energy delivered by the laser could kill harboring viruses in the infected area thereby promoting the healing process and providing relief in pain.

## Description

### FIELD OF INVENTION

The present invention generally relates to laser therapy, more particularly, the present invention relates to treatment of cold sores using the laser therapy.

### BACKGROUND OF INVENTION

At present there is no cure for cold sores but chemotherapy including antivirals and pain killers are often used to hasten the healing and sooth the pain. However, the chemotherapy has more side effects as compared to the benefits.

Laser therapy has been used for treatment of herpes infection i.e. herpes Simplex Virus (cold sores). Laser therapy provides instant relief and promotes healing. It kills the harboring virus and prevents sores. Different kinds of lasers have been developed which may specialize for a particular task. Diode lasers are effective and the output power could be easily adjusted. Water lasers have also been used but they are expensive and release the virus into the air. Since cold sores are caused by herpes species which produce blisters that take two to three weeks to heal, there is a need to investigate the utility of lasers in cold sores to relieve pain and promote healing. The prior art disclosing investigated procedure using laser in treatment of cold sores; includes a U.S. Patent No. 7,730,893**,** entitled *"Electromagnetic radiation therapy",* which discloses an electromagnetic radiation therapy system used in diseases caused by the herpes virus.

Another, WO Patent appl. No. 2001032262**,** entitled *"Miniature cluster laser therapy device",* which discloses a small portable laser therapy device comprising plurality of laser diodes arranged in a cluster along the casing and a source of power to the diodes which can be used to alleviate pain and promote healing; cold-powered laser diodes are used in the device. The device was investigated in treatment herpes infections. Treatment was initiated by using the laser device to provide phototherapy to the spine at the origin of the nerve root. Then each of the specified points was irradiated for two minutes each until the pain subsides.

Another, U.S. Patent appl. No. 20130190742, entitled *"System and method for modification and*/*or smoothing of tissue with laser ablation",* which discloses a laser ablation to modify, smooth, and/or remove necrotic tissue, like bum eschar, decubitus ulcers, and stasis ulcers, and/or remove foreign objects from tissue, in addition to eradicating infectious agents from the tissue being ablated.

### SUMMARY OF THE INVENTION

The present invention, therefore, has as its principal object to provide laser therapy for treatment of herpes simplex infection (cold sores).

Another object of the present invention is to provide a portable and economic laser device.

Yet another object of the present invention is to provide laser therapy that provides relief in pain associated with herpes simplex infection.

Yet another object of present invention is to provide a laser therapy that promotes healing of the herpes simplex infection.

Certain embodiments of current invention, but are not limited to, provides a laser therapy for treatment of herpes simplex infection (cold sores), wherein the therapy provides relief in the pain caused by infection and promote healing of the infected areas. Preferably a diode type lasers could be used wherein the output power could be adjusted according to required power for treatment of the infection.

In addition to the various objects and advantages of the present invention described with some degree of specificity above it should be obvious that additional objects and advantages of the present invention will become more readily apparent to those persons who are skilled in the relevant art from the following more detailed description of the invention, particularly, when such description is taken in conjunction with the attached drawing figures.

### DETAIL DESCRIPTION OF THE INVENTION

Before explaining at least one embodiment of the invention in detail, it is to be understood that the invention is not limited in its application to the details of construction and the arrangements of the components set forth in the following description. The invention is capable of other embodiments and of being practiced and carried out in various ways. Also it is to be understood that the phraseology and terminology employed herein are for the purpose of description and should not be regarded as limiting.

As used in this specification and the appended claims, the singular forms "a", "an", and "the" include plural referents unless the content clearly dictates otherwise.

Unless otherwise defined, all terms (including technical and scientific terms) used herein have the same meaning as commonly understood by one having ordinary skill in the art to which this invention belongs. It will be further understood that terms, such as those defined in commonly used dictionaries, should be interpreted as having a meaning that is consistent with their meaning in the context of the relevant art and the present disclosure and will not be interpreted in an idealized or overly formal sense unless expressly so defined herein.

It is further noted that, unless indicated otherwise, all functions described herein can be performed in either hardware or software, or some combination thereof. In a preferred embodiment, however, the functions are performed by a processor such as a computer or an electronic data processor in accordance with code such as computer program code, software, and/or integrated circuits that are coded to perform such functions, unless indicated otherwise.

The present invention provides laser therapy for treatment of herpes simplex virus infection also known as cold sores. The invention uses laser therapy, and preferably diode type lasers could be used on the infected area including rashes. The laser provides immediate relief in pain and promotes the healing process. The energy delivered by the laser could kill harboring viruses in the infected area thereby promoting the healing process and providing relief in pain.

Although other type of lasers could be used, the diode lasers are particularly advantageous in that they are compact and economic. They kill the virus instead of removing it from the skin into air. Moreover, the diode lasers used for current invention could use rechargeable batteries for portability and compact size will allow easy access to infected areas of body.

Those skilled in the art should appreciate that they can readily use the disclosed conception and specific embodiment as a basis for designing or modifying other structures for carrying out the same purposes of the present invention and that such other structures do not depart from the spirit and scope of the invention in its broadest form.

## Claims

1. A laser therapy for use in treating herpes simplex infections called as cold sores.

2. A laser therapy according to claim 1, wherein said laser therapy uses diode lasers.

3. A laser therapy according to claim 1, wherein said laser therapy provides relief in pain caused by the infection.

4. A laser therapy according to claim 1, wherein said laser therapy promote healing process of infection caused by the herpes simplex infection.
